# EUROPEAN PATENT APPLICATION

(11) **EP 4 321 560 A1**
(43) Date of publication of application: **14.02.2024**
(21) Application number: 22825396.9
(22) Date of filing: 20.06.2022
(51) Int. Cl.: C08J 3/12, C08J 3/24, C08J 3/075, C08F 220/06, C08F 2/01, B29B 9/12

(54) **PREPARATION METHOD OF SUPER ABSORBENT POLYMER AND SUPER ABSORBENT POLYMER**

(30) Priority: 18.06.2021 KR 20210079644; 21.06.2021 KR 20210080231
(71) Applicant: LG Chem, Ltd., Seoul 07336 (KR)
(72) Inventor: NAM, Dae Woo, Daejeon 34122 (KR); MIN, Kyunghoon, Daejeon 34122 (KR); LEE, Seul Ah, Daejeon 34122 (KR); BAEK, Seokhyeon, Daejeon 34122 (KR); AHN, Gyunhyeok, Daejeon 34122 (KR)
(74) Representative: Goddar, Heinz J.
(86) International application number: PCT/KR2022/008716
(87) International publication number: WO 2022/265473

(57) **Abstract**

The present disclosure relates to a method for preparing a super absorbent polymer. More specifically, according to the preparation method of a super absorbent polymer of the present disclosure, it is possible to effectively control the initiation and inhibition of polymerization reactions, thereby reducing the content of unreacted monomers in the final product.

## Description

### [TECHNICAL FIELD]

The present disclosure relates to a preparation method of super absorbent polymer. More specifically, it relates to a preparation method of super absorbent polymer that can remarkably reduce the generation of unreacted monomers in the product.

### [BACKGROUND ART]

Super absorbent polymer (SAP) is a synthetic polymer material capable of absorbing moisture from about 500 to about 1,000 times its own weight, and each manufacturer has denominated it as different names such as SAM (Super Absorbency Material), AGM (Absorbent Gel Material) or the like. Such super absorbent polymers started to be practically applied in sanitary products, and now they are widely used for preparation of water retaining soil products for gardening, water stop materials for the civil engineering and construction, sheets for raising seedling, fresh-keeping agents for food distribution fields, materials for poultice or the like.

Such a super absorbent polymer has been widely used mainly in the field of sanitary materials such as diapers or sanitary napkins. In such hygienic materials, the super absorbent polymer is generally contained in a state of being spread in the pulp. In recent years, however, continuous efforts have been made to provide hygienic materials such as diapers having a thinner thickness. As a part of such efforts, the development of so-called pulpless diapers and the like in which the content of pulp is reduced or pulp is not used at all is being actively advanced.

As described above, in the case of hygienic materials in which the content of pulp is reduced or the pulp is not used, a super absorbent polymer is contained at a relatively high ratio and these super absorbent polymer particles are inevitably contained in multiple layers in the hygienic materials. In order for the whole super absorbent polymer particles contained in the multiple layers to absorb liquid such as urine more efficiently, the super absorbent polymer needs to basically exhibit not only high absorption performance but also fast vortex time.

Such a super absorbent polymer is produced by drying, pulverizing and classifying a hydrogel polymer prepared by performing a crosslinking polymerization of a monomer containing a water-soluble ethylenically unsaturated carboxylic acid or a salt thereof, or is made by surface-crosslinking it again.

When the above-mentioned monomer is subjected to a crosslinking polymerization, an appropriate type of polymerization initiator or polymerization inhibitor is used, and the degree of progress of the polymerization reaction is adjusted through the reaction process conditions and the like. Particularly, in order to activate polymerization, a method of removing dissolved oxygen present in a monomer mixture before charging it into a polymerization reactor is known.

In addition, it is necessary to accurately adjust the initiation or inhibition of the reaction for excellent physical properties of the super absorbent polymer finally prepared, but there is a problem that due to the nature of the radical reaction, this is very difficult, and the physical properties of the super absorbent polymer are deteriorated depending on the amount of polymerization initiator or polymerization inhibitor used.

In particular, the polymerization reaction starts as soon as the initiator and the monomer meet, and the polymerization starts in a transfer line such as a pipe rather than a polymerization reactor, making continuous operation difficult, which may cause a problem that rather the content of unreacted monomer in the final product increases.

Therefore, research is needed to efficiently control the initiation and inhibition of the polymerization reaction.

### [DETAILED DESCRIPTION OF THE INVENTION]

### [Technical Problem]

It is an object of the present disclosure to provide a method for preparing a super absorbent polymer that can efficiently control the initiation and inhibition of polymerization reaction when polymerization proceeds.

### [Technical Solution]

Provided herein is a method for preparing a super absorbent polymer, comprising the steps of: polymerizing a monomer composition containing a water-soluble ethylenically unsaturated monomer having an acidic group, an internal crosslinking agent, and a polymerization initiator to form a polymer in which the water-soluble ethylenically unsaturated monomer having an acid group and the internal crosslinking agent are subjected to a crosslinking polymerization (step 1); neutralizing at least a part of the acidic group of the polymer to form a hydrogel polymer (step 2); micronizing the polymer in the presence of a surfactant (step 3); and drying the neutralized and micronized polymer to prepare dried super absorbent polymer particles (step 4), wherein in the step of forming the polymer, a first monomer composition containing the monomer and the internal crosslinking agent is transferred through a monomer transfer line, the polymerization initiator is transferred through an initiator transfer line, and the monomer transfer line and the initiator transfer line are combined just before being charged into the polymerization reactor, and the first monomer composition and the initiator are mixed to form a second monomer composition.

Also provided herein is a super absorbent polymer prepared by the method for preparing a super absorbent polymer.

### [Advantageous Effects]

According to the preparation method of a super absorbent polymer of the present disclosure, it is possible to effectively control the initiation and inhibition of polymerization reactions, thereby reducing the content of unreacted monomers in the final product

### [DETAILED DESCRIPTION OF THE EMBODIMENTS]

The terms used herein are for the purpose of describing exemplary embodiments only and are not intended to limit the present disclosure. The singular expressions include a plurality of expressions unless expressly stated otherwise in the context. It will be further understood that the terms "comprises", "provides" and/or "has," as used herein specify the presence of stated features, steps, or components, or combinations thereof, but do not exclude the presence or addition of one or more other features, steps, components, or combinations thereof.

The present disclosure can be variously modified and may have various forms, and thus specific embodiments are illustrated and described in detail below. However, the present disclosure is not limited to the specific embodiments and should be construed as including all the changes, equivalents, and substitutions included in the spirit and scope of the present disclosure.

The present disclosure can be variously modified and may have various forms, and thus specific embodiments are illustrated and described in detail below. However, the present disclosure is not limited to the specific embodiments and should be construed as including all the changes, equivalents, and substitutions included in the spirit and scope of the present disclosure.

Hereinafter, a preparation method of a super absorbent polymer and a super absorbent polymer according to a specific embodiment of the present disclosure are described in more detail.

Prior to the description, the terms used herein are for the purpose of mentioning specific exemplary embodiments only and are not intended to limit the present disclosure. And, the singular expressions used herein include a plurality of expressions unless expressly stated otherwise in the context.

According to an embodiment of the present disclosure, there is provided a method for preparing a super absorbent polymer, comprising the steps of: polymerizing a monomer composition containing a water-soluble ethylenically unsaturated monomer having an acidic group, an internal crosslinking agent, and a polymerization initiator to form a polymer in which the water-soluble ethylenically unsaturated monomer having an acid group and the internal crosslinking agent are subjected to a crosslinking polymerization (step 1); neutralizing at least a part of the acidic group of the polymer to form a hydrogel polymer (step 2); micronizing the polymer in the presence of a surfactant (step 3); and drying the neutralized and micronized polymer to prepare dried super absorbent polymer particles (step 4), wherein in the step of forming the polymer, a first monomer composition containing the monomer and the internal crosslinking agent is transferred through a monomer transfer line, the polymerization initiator is transferred through an initiator transfer line, and the monomer transfer line and the initiator transfer line are combined just before being charged into the polymerization reactor, and the first monomer composition and the initiator are mixed to form a second monomer composition.

The term "polymer" or "high molecule" as used herein means a state where a water-soluble ethylenically unsaturated monomer is polymerized, and may include all water content ranges, or all particle size ranges.

Further, the term "super absorbent polymer" means a crosslinked polymer, or a base polymer in the form of powder made of super absorbent polymer particles in which the crosslinked polymer is pulverized, according to the context, or it is used to include those made in a state suitable for the productization by subjecting the crosslinked polymer or base polymer to additional processes, for example, drying, pulverization, classifying, surface crosslinking, etc.

Further, the term "fines" means particles having a particle size of 150 *µ*m or less among the super absorbent polymer particles. The particle size of the polymer particles may be measured according to the European Disposables and Nonwovens Association standard EDANA WSP 220.3.

Further, the term "chopping" refers to cutting the hydrogel polymer into small pieces with a millimeter unit in order to increase the drying efficiency, and is used in distinction from being pulverized to a normal particle level.

Further, the term "micronizing (micronization)" refers to pulverizing the hydrogel polymer to a particle size from several tens to several hundreds of micrometers, and is used in distinction from "chopping".

Conventionally, super absorbent polymers have been prepared by a method including the following steps:
(polymerization) performing a crosslinking polymerization of a water-soluble ethylenically unsaturated monomer having an acid group of which at least a part is neutralized in the presence of an internal crosslinking agent and a polymerization initiator to form a hydrogel polymer;
(chopping) chopping the hydrogel polymer;
(drying) drying the chopped hydrogel polymer; and
(pulverizing/classifying) classifying the dried polymer into normal particles and fines after pulverization.

Among the series of preparation processes as described above, in the polymerization step, the polymerization reaction can proceed as soon as the monomer and the initiator meet. For example, when the monomer and initiator components meet in the transfer line for supplying each reactive material to the reactor, a polymerization reaction proceeds inside the transfer line, which may cause a problem that the transfer line is closed.

In this regard, the present inventors have found that when conducting batch polymerization in the polymerization step, a first monomer composition including the monomer and the internal crosslinking agent is transferred through a monomer transfer line, the polymerization initiator is transferred through an initiator transfer line, the monomer transfer line and the initiator transfer line are combined immediately before being charged into a polymerization reactor, and the first monomer composition and the initiator are mixed to form a second monomer composition, whereby the issues that causes closure of the transport line can be solved. The present disclosure has been completed on the basis of such findings.

Meanwhile, a method of charging a surfactant in order to lower the adhesiveness of the hydrogel polymer in the chopping step has been proposed. However, when a surfactant is charged into the chopping step, there is a problem that due to the high water content of the hydrogel polymer, the surfactant permeates inside the hydrogel polymer instead of existing at the interface of the hydrogel polymer, so that the surfactant cannot properly perform its role.

Therefore, since the chopped particles form particles of several mm or several cm level as compared to the polymer before chopping, the surface area may be increased to some extent, but it is difficult to expect the effect enough to effectively improve the vortex time. In this regard, in order to improve the vortex time, a method of increasing the surface area by further increasing the mechanical force and kneading in the chopping step can be considered, but in this case, aggregation occurs excessively due to the stickiness peculiar to the polymer, and amorphous single particles having irregularities only on the particle surface are formed after chopping, drying and pulverization, and the content of water-soluble components can rather increase due to excessive mixing or kneading.

The present inventors have conducted extensive research to solve the above-mentioned problems, and as a result, found that, unlikely a conventional preparation method of a super absorbent polymer which performs a polymerization in a state in which the acidic group of the water-soluble ethylenically unsaturated monomer is neutralized, when first performing a polymerization in a state where the acidic groups are not neutralized to form a polymer, micronizing the hydrogel polymer in the presence of a surfactant and then neutralizing the acid groups of the polymer, or when neutralizing the acidic groups of the polymer to form a hydrogel polymer and then micronizing the hydrogel polymer in the presence of a surfactant, or when neutralizing the acidic groups present in the polymer simultaneously with the micronizing, the surfactant is present in a large amount on the surface of the polymer and the high adhesiveness of the polymer is lowered, thereby capable of sufficiently performing the role of preventing the polymer from being excessively aggregated and adjusting the aggregation state to a desired level.

Therefore, the polymer is prepared into secondary particles in a form in which primary particles are aggregated, and then pulverization and drying processes are performed under mild conditions, thereby capable of remarkably reducing the amount of fines generated during the process.

Further, when the polymer is micronized in the presence of the surfactant, the hydrophobic functional group portion contained in the surfactant imparts hydrophobicity to the surface of the pulverized super absorbent polymer particles to alleviate a frictional force between particles and increase an apparent density of the super absorbent polymer, and the hydrophilic functional group portion contained in the surfactant can also be bonded to the super absorbent polymer particles to prevent a surface tension of the resin from being lowered. Thereby, the super absorbent polymer prepared according to the above-mentioned preparation method can have a higher apparent density value while exhibiting the same level of surface tension as compared to a resin that does not use a surfactant.

In addition, when polymerization is first performed in a non-neutralized state to form a polymer and then the acidic groups present in the polymer is neutralized, longer chain polymer can be formed and the crosslinking is incomplete, so that the effect of reducing the content of water-soluble components present in a non-crosslinked state can be achieved.

The water-soluble component has the property of being easily eluted when the super absorbent polymer comes into contact with a liquid. Therefore, if the content of water-soluble components is high, most of the dissolved water-soluble components remain on the surface of the super absorbent polymer, which makes the super absorbent polymer sticky and causes a decrease in liquid permeability. Therefore, it is important to keep the content of water-soluble components low from the viewpoint of liquid permeability.

According to one embodiment of the present disclosure, as the polymerization is performed in a non-neutralized state, the content of water-soluble components is lowered, thereby capable of improving the liquid permeability of the super absorbent polymer.

Moreover, the super absorbent polymer prepared according to an embodiment of the present disclosure can have a uniform particle size distribution, thereby providing a super absorbent polymer that is excellent in various absorption properties such as centrifuge retention capacity and absorbency under pressure, a rewet property, a vortex time, and the like.

Hereinafter, the preparation method of the super absorbent polymer according to one embodiment will be described in more detail for each step.

### Step 1: Polymerization Step

First, a monomer composition containing a water-soluble ethylenically unsaturated monomer having an acidic group, an internal crosslinking agent, and a polymerization initiator is polymerized to form a polymer in which the water-soluble ethylenically unsaturated monomer having an acid group and the internal crosslinking agent are subjected to a crosslinking polymerization.

The above step may include a step of mixing a water-soluble ethylenically unsaturated monomer having an acid group, an internal crosslinking agent, and a polymerization initiator to prepare a monomer composition, and a step of polymerizing the monomer composition to form a polymer.

And, the step of forming the polymer is performed by continuous batch polymerization.

The water-soluble ethylenically unsaturated monomer can be any monomer commonly used for the preparation of a super absorbent polymer. As a non-limiting example, the water-soluble ethylenically unsaturated monomer can be a compound represented by the following Chemical Formula 1:

[Chemical Formula 1] R-COOM'

wherein, in Chemical Formula 1,
R is an alkyl group having 2 to 5 carbon atoms containing an unsaturated bond, and
M' is a hydrogen atom, a monovalent or divalent metal, an ammonium group or an organic amine salt.

Preferably, the monomer may be one or more selected from the group consisting of (meth)acrylic acid, and monovalent (alkali) metal salts, divalent metal salts, ammonium salts and organic amine salts of these acids.

When a (meth)acrylic acid and/or a salt thereof is used as the water-soluble ethylenically unsaturated monomer in this way, a super absorbent polymer having improved water absorptivity can be obtained, which is thus advantageous. In addition, as the monomer, maleic anhydride, fumaric acid, crotonic acid, itaconic acid, 2-acryloyl ethane sulfonic acid, 2-methacryloyl ethane sulfonic acid, 2-(meth)acryloyl propane sulfonic acid, or 2-(meth)acrylamide-2-methylpropane sulfonic acid, (meth)acrylamide, N-substituted (meth)acrylate, 2-hydroxyethyl(meth)acrylate, 2-hydroxypropyl(meth)acrylate, methoxypolyethyleneglycol(meth)acrylate, poly ethyleneglycol(meth)acrylate, (N,N)-dimethylaminoethyl(meth)acrylate, (N,N)-dimethylaminopropyl(meth)acrylamide, and the like can be used.

Here, the water-soluble ethylenically unsaturated monomer has an acidic group. As described above, in the preparation of the conventional super absorbent polymer, a hydrogel polymer was formed by performing a crosslinking polymerization of a monomer in which at least a part of the acidic groups are neutralized by a neutralizing agent. Specifically, in the step of mixing the water-soluble ethylenically unsaturated monomer having an acidic group, an internal crosslinking agent, a polymerization initiator and a neutralizing agent, at least a part of the acidic group of the water-soluble ethylenically unsaturated monomer were neutralized.

However, according to one embodiment of the present disclosure, polymerization is first performed in a state in which the acid group of the water-soluble ethylenically unsaturated monomer is not neutralized, thereby forming a polymer.

The water-soluble ethylenically unsaturated monomer (e.g., acrylic acid) in a state in which the acidic group is not neutralized is in a liquid state at room temperature, and has high miscibility with a solvent (water), and thus exists as a mixed solution in the monomer composition. However, the water-soluble ethylenically unsaturated monomer in which the acidic group is neutralized is in a solid state at room temperature, has different solubility depending on the temperature of the solvent (water), and has lower solubility as the temperature is lower.

The water-soluble ethylenically unsaturated monomer in a state in which the acidic group is not neutralized has a higher solubility or miscibility in a solvent (water) than the monomer in which the acidic group is neutralized, and does not precipitate even at a low temperature, so it is advantageous for performing a polymerization at a low temperature for a long time. Therefore, the water-soluble ethylenically unsaturated monomer in which the acidic group is not neutralized can be polymerized for a long period of time to stably form a polymer having a higher molecular weight and a uniform molecular weight distribution.

In addition, longer chain polymer can be formed and the polymerization and crosslinking are incomplete, thereby achieving the effect of reducing the content of water-soluble components present in a non-crosslinked state.

Further, in this manner, when first performing a polymerization in a state where the acidic groups are not neutralized to form a polymer and micronizing the hydrogel polymer in the presence of a surfactant after neutralization, or when micronizing the hydrogel polymer in the presence of a surfactant and neutralizing it, or when neutralizing the acidic groups present in the polymer simultaneously with the micronizing, the surfactant is present in a large amount on the surface of the polymer, thereby capable of sufficiently performing the role of reducing the adhesiveness of the polymer.

The concentration of the water-soluble ethylenically unsaturated monomer in the monomer composition can be appropriately adjusted in consideration of polymerization time and reaction conditions, and may be about 20 to about 60 wt.%, or about 20 to about 40 wt.%.

The term 'internal crosslinking agent' as used herein is a term used to distinguish from a surface crosslinking agent that crosslinks the surface of the super absorbent polymer particles, which will be described later, and serves to introduce a crosslinking bond between the unsaturated bonds of the above-mentioned water-soluble ethylenically unsaturated monomers to form a polymer containing a crosslinked structure.

The crosslinking in the above step proceeds irrespective of the surface or the interior, but when the surface crosslinking step of the super absorbent polymer particles described later proceeds, the surface of the finally prepared super absorbent polymer particles may include a structure newly crosslinked by a surface crosslinking agent, and the interior of the super absorbent polymer particles can allow a structure crosslinked by the internal crosslinking agent to maintain without modification.

According to one embodiment of the disclosure, the internal crosslinking agent may include any one or more of a polyfunctional acrylate-based compound, a polyfunctional allyl-based compound, or a polyfunctional vinyl-based compound.

Non-limiting examples of the polyfunctional acrylate-based compound include ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, tripropylene glycol di(meth)acrylate, polypropylene glycol di(meth)acrylate, butanediol di(meth)acrylate, butylene glycol di(meth)acrylate, hexanediol di(meth)acrylate, pentaerythritol di(meth)acrylate, pentaerythritol tri(meth)acrylate, pentaerythritol tetra(meth)acrylate, dipentaerythritol di(meth)acrylate, dipentaerythritol tri(meth)acrylate, dipentaerythritol tetra(meth)acrylate, dipentaerythritol penta(meth)acrylate, trimethylolpropane di(meth)acrylate, trimethylolpropane tri(meth)acrylate, glycerin di(meth)acrylate, glycerin tri(meth)acrylate, and the like. These may be used alone or in combination of two or more.

Non-limiting examples of the polyfunctional allyl-based compound include ethylene glycol diallyl ether, diethylene glycol diallyl ether, triethylene glycol diallyl ether, tetraethylene glycol diallyl ether, polyethylene glycol diallyl ether, propylene glycol diallyl ether, tripropylene glycol diallyl ether, polypropylene glycol diallyl ether, butanediol diallyl ether, butylene glycol diallyl ether, hexanediol diallyl ether, pentaerythritol diallyl ether, pentaerythritol triallyl ether, pentaerythritol tetraallyl ether, dipentaerythritol diallyl ether, dipentaerythritol triallyl ether, dipentaerythritol tetraallyl ether, dipentaerythritol pentaallyl ether, trimethylolpropane diallyl ether, trimethylolpropane triallyl ether, glycerin diallyl ether, glycerin triallyl ether, and the like. These may be used alone or in combination of two or more.

Non-limiting examples of the polyfunctional vinyl-based compound include ethylene glycol divinyl ether, diethylene glycol divinyl ether, triethylene glycol divinyl ether, tetraethylene glycol divinyl ether, polyethylene glycol divinyl ether, propylene glycol divinyl ether, tripropylene glycol divinyl ether, polypropylene glycol divinyl ether, butanediol divinyl ether, butylene glycol divinyl ether, hexanediol divinyl ether, pentaerythritol divinyl ether, pentaerythritol trivinyl ether, pentaerythritol tetravinyl ether, dipentaerythritol divinyl ether, dipentaerythritol trivinyl ether, dipentaerythritol tetravinyl ether, dipentaerythritol pentavinyl ether, trimethylolpropane divinyl ether, trimethylolpropane trivinyl ether, glycerin divinyl ether, glycerin trivinyl ether, and the like. These may be used alone or in combination of two or more. Preferably, pentaerythritol triallyl ether can be used.

In the above-mentioned polyfunctional allyl-based compound or polyfunctional vinyl-based compound, two or more unsaturated groups contained in the molecule can combine with an unsaturated bond of the water-soluble ethylenically unsaturated monomer or an unsaturated bond of the other internal crosslinking agent to form a crosslinked structure during polymerization process, and unlike acrylate-based compounds containing an ester bond (-(C=O)O-) in the molecule, crosslinking can be more stably maintained even during the neutralization process after the above-mentioned polymerization reaction.

Thereby, the gel strength of the prepared super absorbent polymer is increased, the process stability can be enhanced during the discharge process after polymerization, and the content of water-soluble components can be minimized.

Crosslinking polymerization of the water-soluble ethylenically unsaturated monomer in the presence of such an internal crosslinking agent can be performed in the presence of a polymerization initiator, and if necessary, a thickener, a plasticizer, a storage stabilizer, an antioxidant, and the like.

In the monomer composition, the internal crosslinking agent may be used in an amount of 0.01 to 5 parts by weight with respect to 100 parts by weight of the water-soluble ethylenically unsaturated monomer. For example, the internal crosslinking agent may be used in an amount of 0.01 parts by weight or more, or 0.05 parts by weight or more, or 0.1 parts by weight or more, and 5 parts by weight or less, or 3 parts by weight or less, or 2 parts by weight or less, or 1 part by weight or less, or 0.7 parts by weight or less, with respect to 100 parts by weight of the water-soluble ethylenically unsaturated monomer. When the content of the internal crosslinking agent is too low, crosslinking does not occur sufficiently, which may make it difficult to achieve strength above an appropriate level, and when the content of the internal crosslinking agent is too high, the internal crosslinking density is increased, which may make it difficult to realize a desired centrifuge retention capacity.

The polymer formed using such an internal crosslinking agent has a three-dimensional network structure in which main chains formed by polymerizing the water-soluble ethylenically unsaturated monomers are crosslinked by the internal crosslinking agent. When the polymer has a three-dimensional network structure in this way, the centrifuge retention capacity and the absorbency under pressure, which are general physical properties of the super absorbent polymer, can be remarkably improved as compared to the case of a two-dimensional linear structure that is not further crosslinked by an internal crosslinking agent.

According to one embodiment of the present disclosure, the step of polymerizing the monomer composition to form a polymer may be performed in a batch type reactor.

In a conventional method for preparing a super absorbent polymer, the polymerization method is largely classified into the thermal polymerization and the photo-polymerization depending on the polymerization energy source. Usually, the thermal polymerization can be performed in a reactor like a kneader equipped with agitating spindles and the photo-polymerization can be performed in a reactor equipped with a movable conveyor belt or in a container with a flat bottom.

Meanwhile, as the polymerization method as described above generally proceeds in a short polymerization reaction time of about 1 hour or less, the molecular weight of the polymer is not large, and a polymer having a wide molecular weight distribution is formed.

Meanwhile, when the photo-polymerization is performed in a reactor equipped with a movable conveyor belt or in a container with a flat bottom, the morphology of the hydrogel polymer typically obtained may be a sheet-like hydrogel polymer having a width of the belt, and the thickness of the polymer sheet may vary depending on the concentration of the monomer composition supplied thereto and the supply speed or the supply amount, but it is usually obtained to have a thickness of about 0.5 to about 5 cm.

However, if the monomer composition is supplied to the extent that the thickness of the sheet-like polymer becomes too thin, the production efficiency becomes low, which is not preferred. If the thickness of the sheet-like polymer is made too thick for production, the polymerization reaction may not uniformly occur over the entire thickness, which makes it difficult to form high-quality polymer.

In addition, in the polymerization in a reactor having a reactor agitation shaft and equipped with a conveyor belt, a new monomer composition is supplied to the reactor while the polymerization result is moving, and polymerization is performed in a continuous mode, so that polymers having different polymerization rates are mixed, whereby polymerization is hard to uniformly occur in the entire monomer composition, and deterioration of overall physical properties may occur.

However, according to one embodiment of the present disclosure, as polymerization proceeds by a fixed-bed type in a batch reactor, polymers having different polymerization rates are less likely to be mixed, and thus a polymer having uniform quality can be obtained.

Further, the polymerization step is performed in a batch reactor having a predetermined volume, and the polymerization reaction is performed for a longer period of time, for example, 6 hours or more, than when polymerization is continuously performed in a reactor equipped with a conveyor belt. Despite the long polymerization reaction time as described above, since polymerization is performed for the water-soluble ethylenically unsaturated monomer in a non-neutralized state, the monomer is not easily precipitated even if the polymerization is performed for a long period of time, which is advantageous for performing a polymerization for a long period of time.

Meanwhile, as the polymerization in the batch reactor of the present disclosure utilizes a thermal polymerization method, a thermal polymerization initiator is used as the polymerization initiator.

As the thermal polymerization initiator, one or more compounds selected from the group consisting of a persulfate-based initiator, an azo-based initiator, hydrogen peroxide, and ascorbic acid may be used. Specific examples of the persulfate-based initiator may include sodium persulfate (Na₂S₂O₈), potassium persulfate (K₂S₂O₈), ammonium persulfate (NH₄)₂S₂O₈), and the like. Further, examples of the azo-based initiator may include 2,2-azobis-(2-amidinopropane)dihydrochloride, 2,2-azobis-(N,N-dimethylene)isobutyramidine dihydrochloride, 2-(carbamoylazo)isobutylonitrile, 2,2-azobis[2-(2-imidazolin-2-yl)propane]dihydrochloride, 4,4-azobis-(4-cyanovaleric acid) and the like. More various thermal polymerization initiators are well disclosed in "Principle of Polymerization" written by Odian, (Wiley, 1981), p203, which may be incorporated herein by reference.

If the concentration of the polymerization initiator is too low, the polymerization speed may be slowed down and thus a large amount of residual monomers may be extracted from the final product, which is not preferable. On the contrary, if the concentration of the polymerization initiator is too high, a polymer chain forming a network may become short, and thus, the physical properties of polymer may be degraded such as increase in the content of water-soluble components and decrease in absorbency under pressure, which is not preferable.

The amount of the thermal polymerization initiator used may affect the physical properties of the base polymer prepared through the subsequent process, and particularly, affect the content of water-soluble components of the base polymer. When the content of water-soluble components becomes high, the physical properties of the final prepared super absorbent polymer deteriorate, and particularly, the absorbency under pressure (AUP) and liquid permeability deteriorate. In addition, when the thermal polymerization initiator is used in an excessively small amount, the efficiency of hydrogel polymerization may decrease, and thus various physical properties of the finally prepared super absorbent polymer may be deteriorated.

Generally, the polymerization initiators described above are used in a form of being initially included in the first monomer composition (mixture) containing a water-soluble ethylenically unsaturated monomer and an internal crosslinking agent, but according to one aspect of the present disclosure, the initiator is prepared separately from the above-mentioned first monomer composition.

Specifically, in the step of forming the polymer, the first monomer composition containing the monomer and the internal crosslinking agent is transferred through a monomer transfer line, the polymerization initiator is transferred through an initiator transfer line, the monomer transfer line and the initiator transfer line are combined just before being charged into the polymerization reactor, and the first monomer composition and the initiator are mixed to form a second monomer composition.

According to the method as above, it is possible to prevent the problem that the polymerization reaction is initiated in the transfer line to which the polymerization reactant is supplied and thus the polymerization line is closed.

And, in the step of combining the monomer transfer line and the initiator transfer line, a ratio (speed ratio) of the supply speed (m/s) of the initiator supplied from the initiator transfer line to the supply speed (m/s) of the first monomer mixture supplied from the monomer transfer line may be, preferably, about 3.6 or more, or about 4.0 or more, or about 5.0 or more, or about 7.0 or more. The upper limit is not significant, but may be about 20 or less, or about 17 or less, or about 15 or less.

The supply speed as above, that is, the linear speed supplied from the transfer line, can be calculated by measuring the mass and density (kg/hr; kg/m³) or volume (m³/hr) supplied per unit time, and using the cross sectional area of the transfer line.

That is, it should be noted that the above speed ratio is not the speed related to the supply amount at the time of supply, but the ratio to the linear speed in each transfer line.

When two fluids are mixed adjacent to each other, according to Bernoulli's principle, the fluid side with a relatively low speed (e.g., monomer transfer line) is increased in pressure and the fluid side with a relatively high speed (e.g., initiator transfer line) is reduced in pressure.

Mixing takes place while the substances contained in each fluid are diffused by the pressure difference between the two, but when the speed range as described above is satisfied, rapid diffusion occurs instantaneously, and the monomer component and the initiator component can be quickly and uniformly mixed.

Thereby, the polymerization reaction can be prevented from being initiated in the transfer line, and at the same time, the monomer component and the initiator component are uniformly mixed, so that the polymerization reaction inside the reactor can also proceed uniformly as a whole, thereby remarkably reducing the unreacted monomer component in the prepared polymer.

Then, in the step of combining the monomer transfer line and the initiator transfer line, the ratio (flow rate ratio) of the supply flow rate (kg/hr) of the initiator supplied from the initiator transfer line to the supply flow rate (kg/hr) of the first monomer mixture supplied from the monomer transfer line may be about 0.01 to about 0.1.

Meanwhile, in one embodiment of the present disclosure, the polymerization may be initiated by adding the initiator and a reducing agent forming a redox couple together.

Specifically, when the initiator and the reducing agent are charged into the polymer solution, they react with each other to form radicals.

The formed radicals react with the monomers, and the oxidation-reduction reaction between the initiator and the reducing agent is highly reactive. Thus, even if only a small amount of an initiator and a reducing agent are added, polymerization is initiated and the process temperature does not need to be increased, so that low-temperature polymerization is possible and changes in physical properties of the polymer solution can be minimized.

The polymerization reaction using the oxidation-reduction reaction may occur smoothly even at a temperature near or below room temperature (25°C). As an example, the polymerization reaction can be performed at a temperature of 5°C or more and 25°C or less, or 5°C or more and 20°C or less.

In one embodiment of the present disclosure, when a persulfate-based initiator is used as the initiator, the reducing agent may include at least one selected from the group consisting of sodium metabisulfite (Na₂S₂O₅); tetramethyl ethylenediamine (TMEDA); a mixture of iron(II) sulfate and EDTA (FeSO₄/EDTA); sodium formaldehyde sulfoxylate; and disodium 2-hydroxy-2-sulfinoacteate.

In one example, potassium persulfate may be used as an initiator, and disodium 2-hydroxy-2-sulfinoacetate may be used as a reducing agent; or ammonium persulfate may be used as an initiator and tetramethylethylenediamine may be used as a reducing agent; or sodium persulfate may be used as an initiator, and sodium formaldehyde sulfoxylate may be used as a reducing agent.

In another embodiment of the present disclosure, when a hydrogen peroxide-based initiator is used as the initiator, the reducing agent may include at least one selected from the group consisting of ascorbic acid; sucrose; sodium sulfite (Na₂SO₃); sodium metabisulfite (Na₂S₂O₅); tetramethyl ethylenediamine (TMEDA); a mixture of iron(II) sulfate and EDTA (FeSO₄/EDTA); sodium formaldehyde sulfoxylate; disodium 2-hydroxy-2-sulfinoacteate; and disodium 2-hydroxy-2-sulfoacteate.

That is, the second monomer composition further includes a reducing agent, and the reducing agent may be supplied together with the initiator through the initiator transfer line, or may be supplied through a separate reducing agent transfer line.

And, the ratio (speed ratio) of the supply speed (m/s) of the reducing agent to the supply speed (m/s) of the first monomer mixture supplied from the monomer transfer line may be about 3.5 or more, or about 4.0 or more, or about 5.0 or more, or about 6.0 or more, or about 6.5 or more, and the upper limit is not significant, but may be about 20 or less, or about 17 or less, or about 15 or less.

The technical significance of the supply speed ratio of the reducing agent is replaced by the explanation of the supply speed ratio of the initiator.

The monomer composition may further contain additives such as a thickener, a plasticizer, a storage stabilizer, and an antioxidant, if necessary.

And, the monomer composition containing the monomer may be, for example, in a solution state dissolved in a solvent such as water, and the solid content in the monomer composition in a solution state, that is, the concentration of the monomer, the internal crosslinking agent and the polymerization initiator may be appropriately adjusted in consideration of the polymerization time, reaction conditions and the like. For example, the solids content in the monomer composition may be 10 to 80% by weight, or 15 to 60 wt.%, or 30 to 50 wt.%.

The solvent that can be used at this time can be used without limitations in the constitution as long as it is able to dissolve the above-mentioned components, and for examples, one or more in combination selected from water, ethanol, ethylene glycol, diethylene glycol, triethylene glycol, 1,4-butanediol, propylene glycol, ethylene glycol monobutyl ether, propylene glycol monomethyl ether, propylene glycol monomethyl ether acetate, methyl ethyl ketone, acetone, methyl amyl ketone, cyclohexanone, cyclopentanone, diethylene glycol monomethyl ether, diethylene glycol ethylether, toluene, xylene, butyrolactone, carbitol, methyl cellosolve acetate and N,N-dimethylacetamide can be used.

As the polymer obtained by such a method is polymerized using the ethylenically unsaturated monomer in a non-neutralized state, a polymer having a high molecular weight and a uniform molecular weight distribution as described above can be formed, and the content of water-soluble components can be reduced.

The polymer obtained by such a method is in the form of a hydrogel polymer, and may have a moisture content of 30 to 80 wt.%. For example, the water content of the polymer may be 30 wt.% or more, or 45 wt.% or more, or 50 wt.% or more, and 80 wt.% or less, or 70 wt.% or less, or 60 wt.% or less.

When the moisture content of the polymer is too low, it is difficult to secure an appropriate surface area in the subsequent pulverization step and thus it may not be possible to pulverize effectively. When the moisture content of the polymer is too high, the pressure received in the subsequent pulverization step increases and thus it may be difficult to pulverize to a desired particle size.

Meanwhile, the "moisture content" as used herein is the content occupied by moisture based on the total weight of a polymer, and it means as a value obtained by subtracting the weight of polymer of a dry state from the weight of the polymer. Specifically, it is defined as a value calculated by measuring the weight loss according to moisture evaporation in the polymer while raising the temperature of polymer of a crumb state through infrared heating to dry. At this time, the drying condition is set up such that the temperature is raised from room temperature to about 180°C and then maintained at 180°C, and the total drying time is 40 minutes including a temperature raising step of 5 minutes.

When a reducing agent is used, the reducing agent may be supplied together with the initiator as the initiator transfer line, and in the step of combining the monomer transfer line and the initiator transfer line, the ratio (speed ratio) of the supply speed of the reducing agent supplied from the initiator transfer line to the supply speed of the first monomer mixture supplied from the monomer transfer line may be 4.0 or more.

In the step of combining the monomer transfer line and the initiator transfer line, the ratio (flow rate ratio) of the supply flow rate of the initiator supplied from the initiator transfer line to the supply flow rate of the first monomer mixture supplied from the monomer transfer line may be about 0.01 to about 0.1.

And, the monomer composition containing the monomer may be, for example, in a solution state dissolved in a solvent such as water, and the solid content in the monomer composition in a solution state, that is, the concentration of the monomer, the internal crosslinking agent and the polymerization initiator may be appropriately adjusted in consideration of the polymerization time, reaction conditions and the like. For example, the solids content in the monomer composition may be 10 to 80 wt.%, or 15 to 60 wt.%, or 30 to 50 wt.%.

The solvent that can be used at this time can be used without limitation in its constitution as long as it can dissolve the above-mentioned components, For example, one or more selected from water, ethanol, ethylene glycol, diethylene glycol, triethylene glycol, 1,4-butanediol, propylene glycol, ethylene glycol monobutyl ether, propylene glycol monomethyl ether, propylene glycol monomethyl ether acetate, methyl ethyl ketone, acetone, methyl amyl ketone, cyclohexanone, cyclopentanone, diethylene glycol monomethyl ether, diethylene glycol ethyl ether, toluene, xylene, butyrolactone, carbitol, methyl cellosolve acetate and N,N-dimethylacetamide, etc.can be used in combination.

The monomer composition may further include additives such as a thickener, a reducing agent, a plasticizer, a preservation stabilizer, and an antioxidant, if necessary.

### Step 2: Neutralization Step and Step 3: Micronizing Step

Next, the step neutralizing at least a part of the acidic group of the polymer (step 2) is performed.

At this time, as the neutralizing agent, a basic material such as sodium hydroxide, potassium hydroxide, ammonium hydroxide and the like that can neutralize an acidic group can be used.

Further, the degree of neutralization, which refers to the degree of being neutralized by a neutralizing agent among the acidic groups contained in the polymer, may be 50 to 90 mol%, or 60 to 85 mol%, or 65 to 85 mol%, or 65 to 75 mol%. The range of the degree of neutralization may vary depending on the final physical properties. If the degree of neutralization is too high, the absorption capacity of the super absorbent polymer is lowered and the concentration of carboxyl groups on the surface of the particles is too low, making it difficult to perform surface crosslinking in a subsequent process, which may reduce absorbency under pressure or liquid permeability. On the contrary, if the degree of neutralization is too low, not only the absorbency of the polymer is greatly reduced, but it can also exhibit properties similar to elastic rubber, which is difficult to handle.

Simultaneously with the second step, or before or after performing the second step, a step of micronizing the polymer in the presence of a surfactant is performed (step 3).

The above step is a step of micronizing the polymer in the presence of a surfactant, which is a step in which the polymer is simultaneously chopped and aggregated to a size of several tens to several hundreds of micrometers, rather than chopping the polymer to a millimeter size. That is, this is a step of preparing secondary aggregated particles in a shape in which primary particles chopped to a size of tens to hundreds of micrometers are aggregated by imparting appropriate adhesiveness to the polymer. The water-containing super absorbent polymer particles, which are secondary aggregated particles prepared through such a step, have a normal particle size distribution and significantly increased the surface area, thereby remarkably improving the vortex time.

After mixing the polymer and the surfactant in this way, the polymer is micronized in the presence of the surfactant. Thereby, secondary aggregated particles chopped and aggregated in a state where the super absorbent polymer particles and the surfactant are mixed, can be produced.

Here, the term "hydrous super absorbent polymer particles" are particles having a moisture content (water content) of about 30 wt.% or more, which are those in which the polymer is chopped and aggregated in the form of particles without a drying process, and thus it may have a moisture content of 30 to 80 wt.%, similarly to the above polymer.

According to one embodiment of the present disclosure, the surfactant may be a compound represented by the following Chemical Formula 2 or a salt thereof, but the present disclosure is not limited thereto: 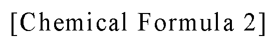 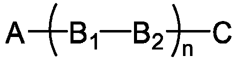 wherein, in Chemical Formula 2,
A is an alkyl having 5 to 21 carbon atoms,
B₁ is -OCO-, -COO-, or -COOCH(R₁)COO-,
B₂ is -CH₂-, -CH₂CH₂-, -CH(R₂)-, -CH=CH-, or -C=C-,
where, R₁ and R₂ are each independently an alkyl having 1 to 4 carbon atoms,
n is an integer of 1 to 3, and
C is a carboxyl group,

At this time, the surfactant is at least one selected from the group consisting of a carboxylic acid represented by Chemical Formula 2 and a metal salt thereof. Specifically, the surfactant is at least one selected from the group consisting of a carboxylic acid represented by Chemical Formula 2, an alkali metal salt of a carboxylic acid represented by Chemical Formula 2, and an alkaline earth metal salt of a carboxylic acid represented by Chemical Formula 2. More specifically, the surfactant is one of the carboxylic acid represented by Chemical Formula 2, an alkali metal salt of the carboxylic acid represented by Chemical Formula 2, and an alkaline earth metal salt of the carboxylic acid represented by Chemical Formula 2.

In Chemical Formula 2, A is a portion exhibiting hydrophobicity and may be a linear or branched alkyl group having 5 to 21 carbon atoms, but the case where A is an alkyl group having a linear structure is more advantageous in terms of inhibiting aggregation of the pulverized particles and improving dispersibility. When A is an alkyl group having less than 5 carbon atoms, there is a problem that the aggregation control of the pulverized particles is not effectively achieved due to the short chain length, and when A is an alkyl group having more than 21 carbon atoms, there may be a problem that the mobility of the surfactant is reduced, so that it is not effectively mixed with the polymer, or the unit price of the composition increases due to a rise in the cost of the surfactant.

Specifically, in Chemical Formula 2, A may be a linear alkyl having 5 to 21 carbon atoms, that is, n-pentyl, n-hexyl, n-heptyl, n-octyl, n-nonyl, n-decanyl, n-undecanyl, n-dodecanyl, n-tridecanyl, n-tetradecanyl, n-pentadecanyl, n-hexadecanyl, n-heptadecanyl, n-octadecanyl, n-nonadecanyl, n-icosanyl, or n-heneicosanyl.

More specifically, A may be a linear alkyl having 6 to 18 carbon atoms. For example, A may be -C₆H₁₃, -C₁₁H₂₃, -C₁₂H₂₅, -C₁₇H₃₅, or -C₁₈H₃₇.

Further, in Chemical Formula 2, the (B₁-B₂) portion is a portion serving to improve the adsorption performance to the polymer surface, which may be lacked in case of the C portion alone. When the carbon number of B₂ is 3 or more, the distance between the B₁ portion and the C portion increases, and the adsorption performance to the polymer may deteriorate.

At this time, R₁ and R₂ may be each independently a linear or branched alkyl having 1 to 4 carbon atoms, and more specifically, R₁ and R₂ may be each independently methyl, ethyl, propyl, isopropyl, butyl, isobutyl, sec-butyl, or tert-butyl, but in terms of the fact that the surfactant is adsorbed to the superabsorbent resin particles, since it is advantageous that the molecular structure of the surfactant is not bulky, both R₁ and R₂ may be methyl.

Further, in Chemical Formula 2, n may be 1, 2, or 3. More specifically, n, meaning the number of (B₁-B₂), is preferably 1, considering that the (B₁-B₂) portion is for enhancing the adsorption performance to the C portion and that the surfactant has a molecular length for being effectively adsorbed to the polymer,

Specifically, in Chemical Formula 2, B₁ may be 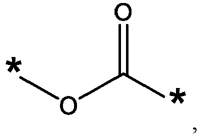 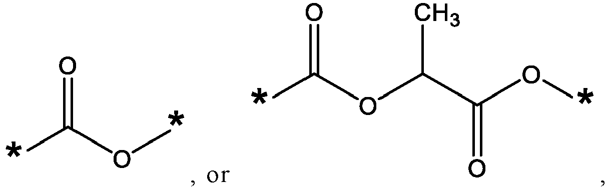 where * is a bonding site with an adjacent atom.

For example, B₁ may be 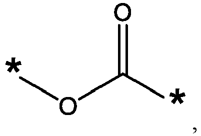 or

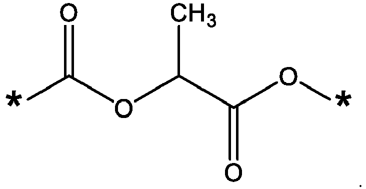

Further, in Chemical Formula 2, B₂ may be 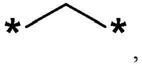 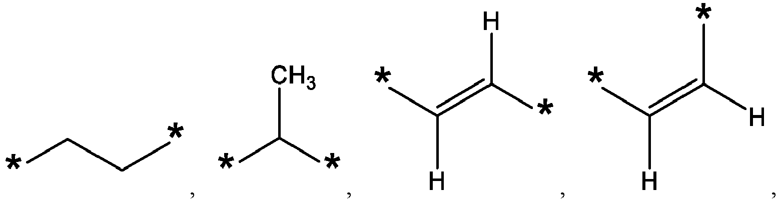 or 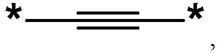 where * is a bonding site with an adjacent atom. At this time, from the viewpoint of improving the adsorption performance of the surfactant to the crosslinked polymer together with the C portion, B₂ is preferably

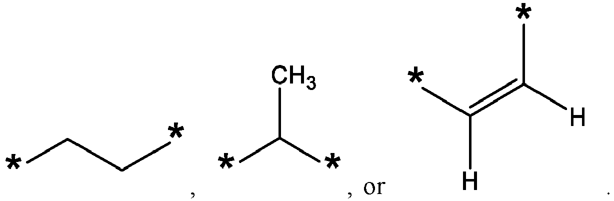

Further, in Chemical Formula 2, the C portion represents a portion exhibiting hydrophilicity, and is a carboxyl group (COOH), provided that when the surfactant is a salt, it is a carboxylate group (COO-).

In other words, the surfactant may be a compound represented by the following Chemical Formula 2a: 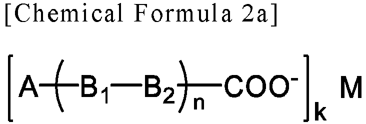 wherein, in Chemical Formula 2a,
M is H+, a monovalent cation of an alkali metal, or a divalent cation of an alkaline earth metal,
k is 1 if M is H+ or a monovalent cation of an alkali metal, and is 2 if M is a divalent cation of an alkaline earth metal,
A, B₁, B₂ and n are the same as defined in Chemical Formula 2.

More specifically, when the surfactant is an alkali metal salt of a carboxylic acid represented by Chemical Formula 2, the surfactant may be represented by the following Chemical Formula 2': 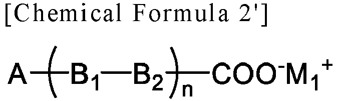 wherein, in Chemical Formula 2',
M₁ is an alkali metal, for example sodium or potassium, and
A, B₁, B₂ and n are the same as defined in Chemical Formula 2.

Further, when the surfactant is an alkaline earth metal salt of a carboxylic acid represented by Chemical Formula 2, the surfactant may be represented by the following Chemical Formula 2": 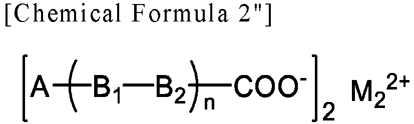 wherein, in Chemical Formula 2", M₂ is an alkaline earth metal, for example, calcium, and
A, B₁, B₂ and n are the same as defined in Chemical Formula 2.

In one example, the surfactant may be any one carboxylic acid selected from the group consisting of:

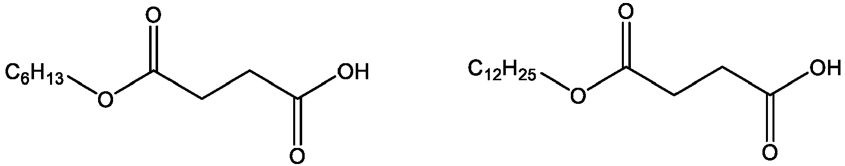

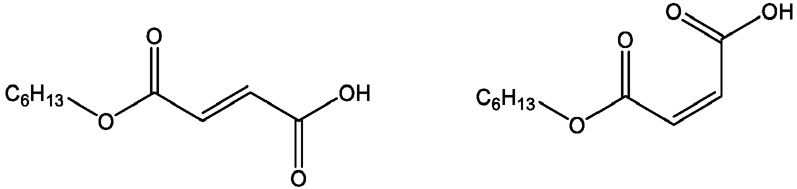

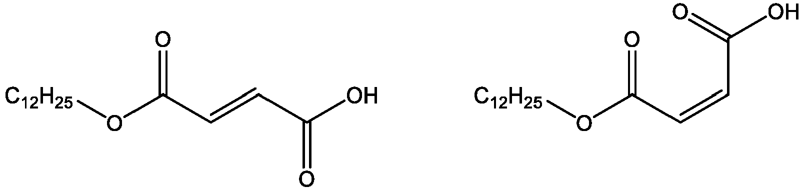

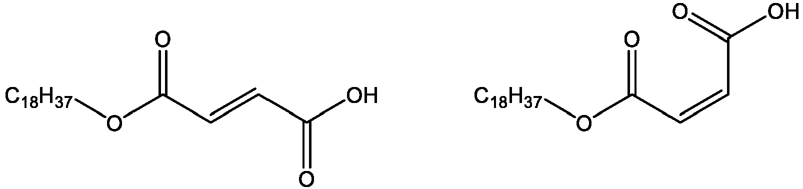

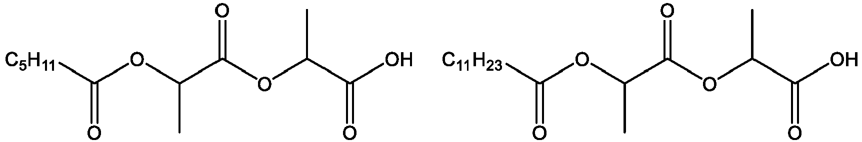

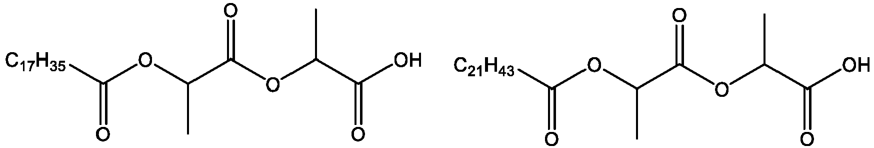

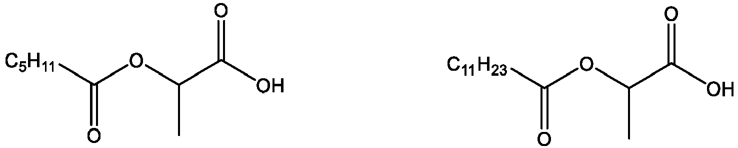

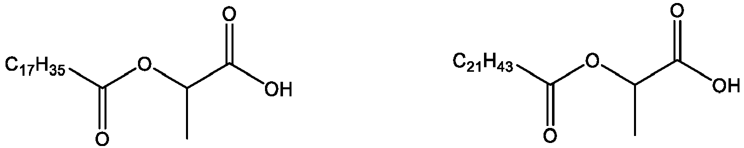

Alternatively, the surfactant may be any one alkali metal salt selected from the group consisting of: 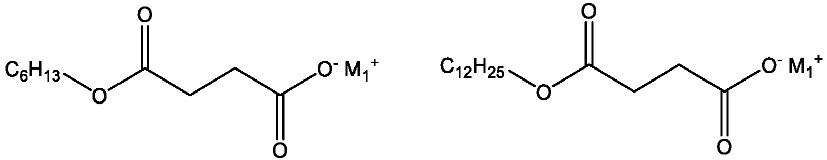 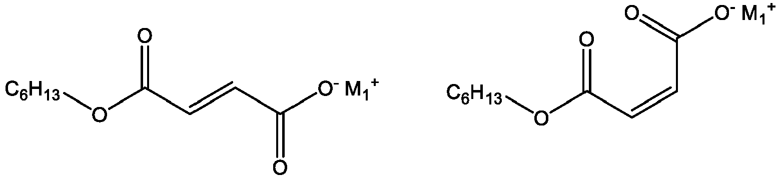 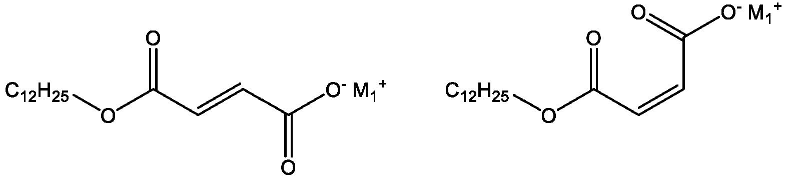 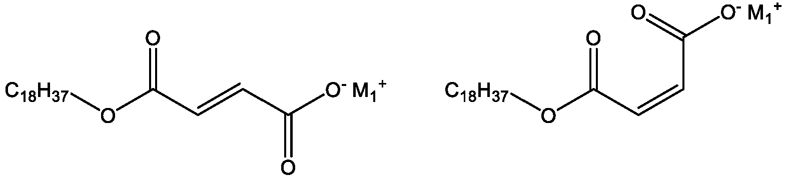 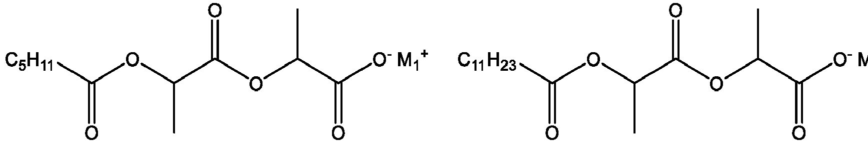 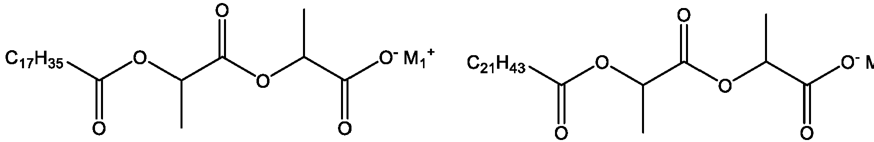 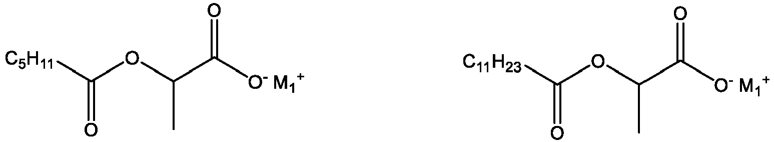 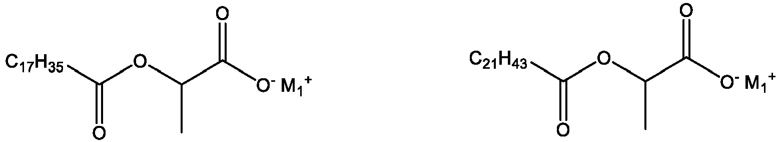 wherein,
each M₁ is independently an alkali metal.

Alternatively, the surfactant may be any one alkaline earth metal salt selected from the group consisting of: 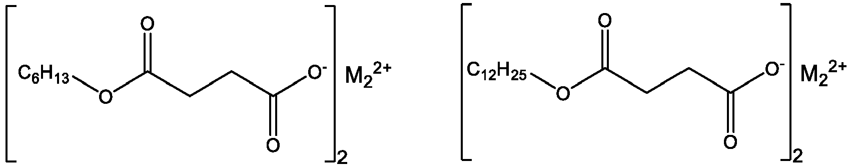 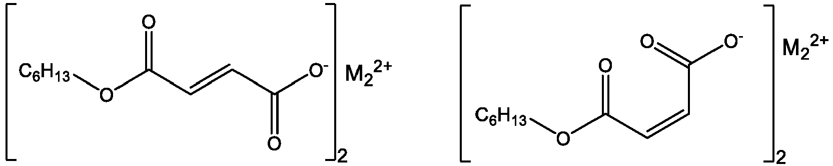 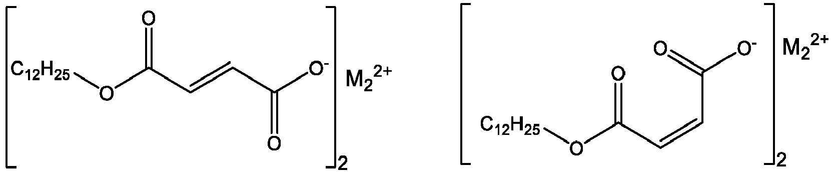 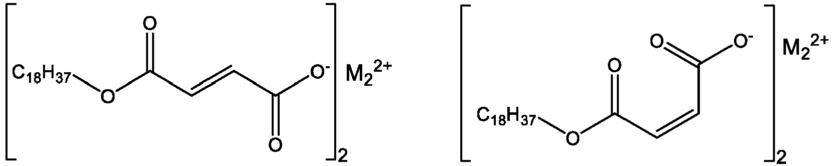 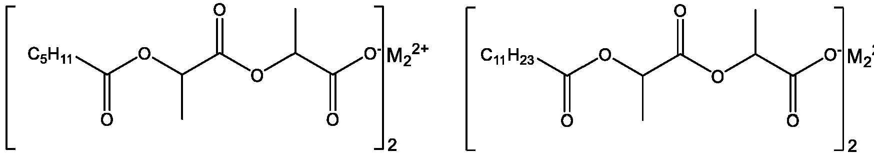 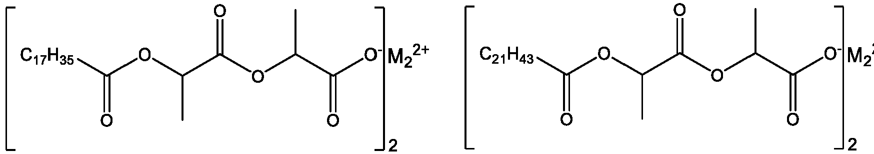 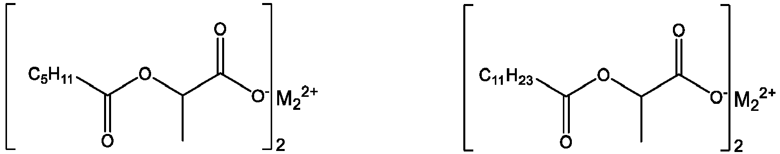 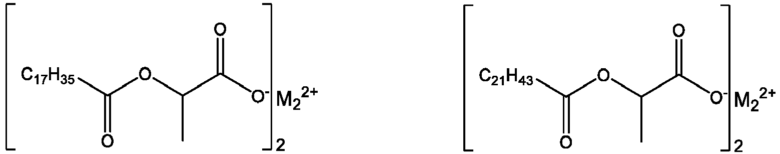 wherein,
each M₂ is independently an alkaline earth metal.

For example, the surfactant may be any one of compounds represented by the following Chemical Formulas 1-1 to 1-7, but is not limited thereto:

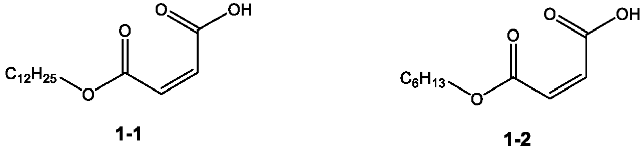

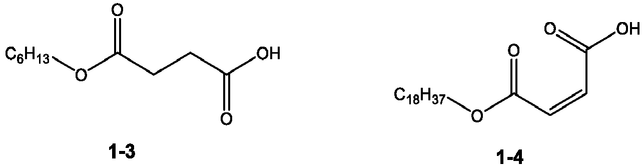

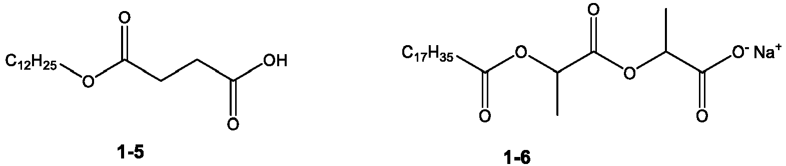

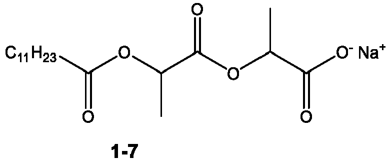

According to another embodiment of the present disclosure, the surfactant may be a compound represented by the following Chemical Formula 3 or a salt thereof, but the present disclosure is not limited thereto: 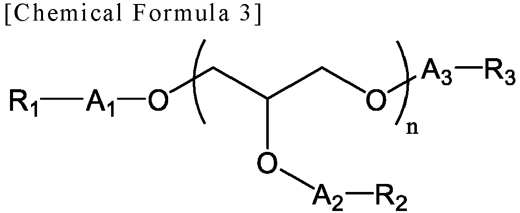 wherein, in Chemical Formula 3,
A₁, A₂ and A₃ are each independently a single bond, carbonyl, 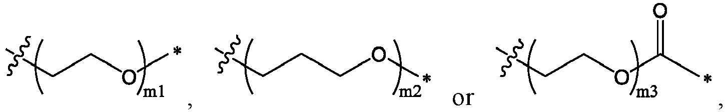 provided that one or more thereof are carbonyl or 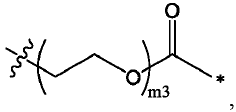 where, m1, m2 and m3 are each independently an integer of 1 to 8, each 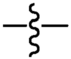 is connected to an adjacent oxygen atom, and 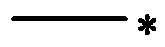 is connected to adjacent R₁, R₂ an R₃, respectively,
R₁, R₂ and R₃ are each independently hydrogen, a linear or branched alkyl having 6 to 18 carbon atoms, or a linear or branched alkenyl having 6 to 18 carbon atoms, and
n is an integer of 1 to 9.

The surfactant is mixed with the polymer and added so that the micronizing step can be easily performed without aggregation phenomenon.

The surfactant represented by Chemical Formula 3 is a nonionic surfactant and has excellent surface adsorption performance due to hydrogen bonding even with non-neutralized polymers, which is thus suitable for achieving a desired aggregation control effect. Meanwhile, in the case of an anionic surfactant instead of a nonionic surfactant, when mixed with a polymer neutralized with a neutralizing agent such as NaOH, Na₂SO₄, it is adsorbed via Na⁺ ions ionized in the carboxyl group substituents of the polymer, and when mixed into a non-neutralized polymer, there is a problem that the efficiency of adsorption to the polymer is relatively low due to competition with the anions of the carboxyl group substituents of the polymer.

Specifically, in the surfactant represented by Chemical Formula 3, the hydrophobic functional group is R₁, R₂ and R₃ portions which are terminal functional groups (if not hydrogen), and the hydrophilic functional group further includes glycerol-derived portions within the chain and terminal hydroxyl groups (n=1 to 3 when Aₙ is a single bond and at the same time, Rₙ is hydrogen), wherein the glycerol-derived portion and the terminal hydroxyl group serve to improve adsorption performance on the polymer surface as a hydrophilic functional group. Thereby, aggregation of the super absorbent polymer particles can be effectively suppressed.

In Chemical Formula 3, R₁, R₂ and R₃ portions which are hydrophobic functional groups (if not hydrogen) are each independently a linear or branched alkyl having 6 to 18 carbon atoms or a linear or branched alkenyl having 6 to 18 carbon atoms. In this case, when R₁, R₂, and R₃ portions (if not hydrogen) are an alkyl or alkenyl having less than 6 carbon atoms, there is a problem that the chain length is short and the aggregation control of the pulverized particles cannot be performed effectively. When R₁, R₂, R₃ portions (if not hydrogen) are alkyl or alkenyl having more than 18 carbon atoms, there may be a problem that the mobility of the surfactant may be reduced and thus, it may not be effectively mixed with the polymer and the unit price of the composition increases due to the increase in the cost of the surfactant.

Preferably, R₁, R₂ and R₃ are hydrogen, or in the case of a linear or branched alkyl having 6 to 18 carbon atoms, it may be 2-methylhexyl, n-heptyl, 2-methylheptyl, n-octyl, n-nonyl, n-decanyl, n-undecanyl, n-dodecanyl, n-tridecanyl, n-tetradecanyl, n-pentadecanyl, n-hexadecanyl, n-heptadecanyl, or n-octadecanyl, or in the case of a linear or branched alkenyl having 6 to 18 carbon atoms, it may be 2-hexenyl, 2-heptenyl, 2-octenyl, 2-nonenyl, n-dekenyl, 2-undekenyl, 2-dodekenyl, 2-tridekenyl, 2-tetradekenyl, 2-pentadekenyl, 2-hexadekenyl, 2-heptadekenyl, or 2-octadekenyl.

The surfactant may be selected from compounds represented by the following Chemical Formulas 3-1 to 3-14:

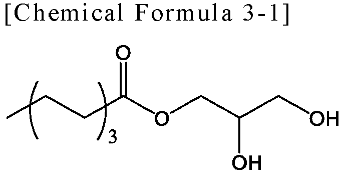

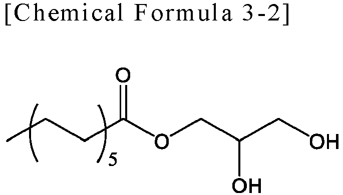

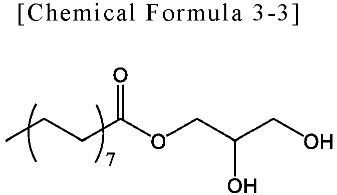

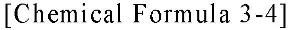

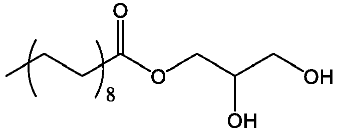

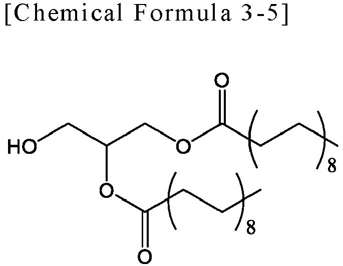

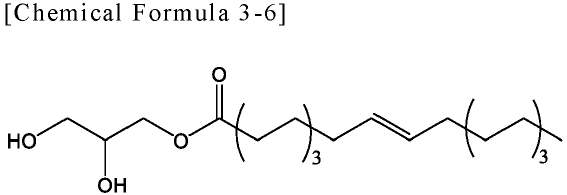

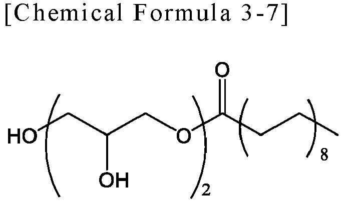

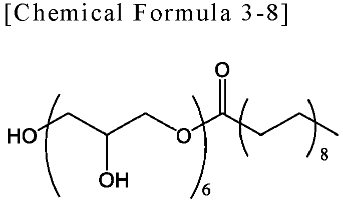

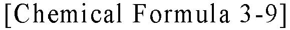

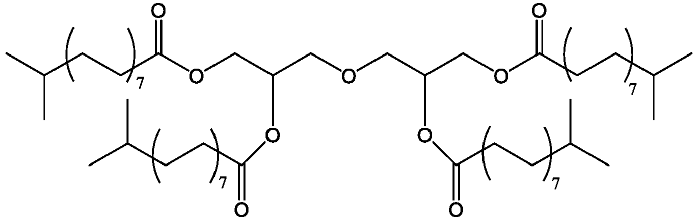

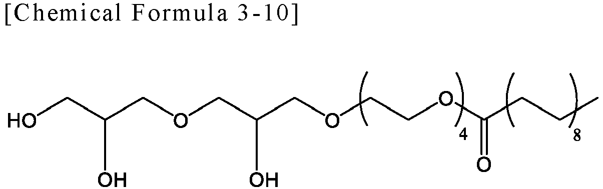

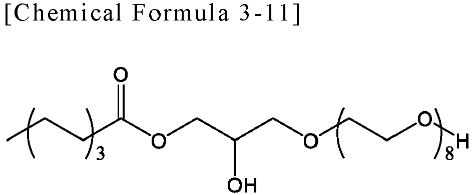

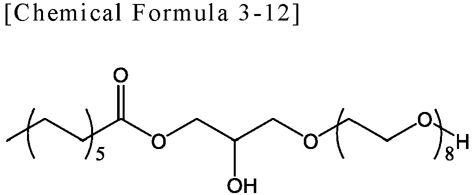

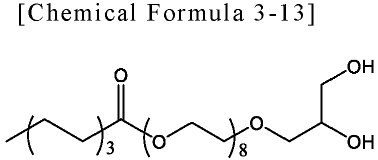

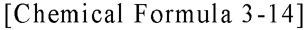

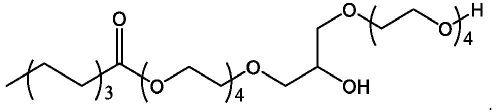

Meanwhile, the surfactant may be used in an amount of 0.01 to 10 parts by weight with respect to 100 parts by weight of the polymer. When the surfactant is used in an excessively small amount, it is not evenly adsorbed on the surface of the polymer and thus, reaggregation of the particles after pulverization may occur. When the surfactant is used in an excessively large amount, the overall physical properties of the finally prepared super absorbent polymer may be deteriorated. For example, the surfactant may be used in an amount of 0.01 parts by weight or more, 0.015 parts by weight or more, or 0.1 parts by weight or more, and 5 parts by weight or less, 3 parts by weight or less, 2 parts by weight or less, or 1 part by weight with respect to 100 parts by weight of the polymer.

The method for mixing such a surfactant with the polymer is not particularly limited as long as it can be uniformly mixed with the polymer, and can be appropriately adopted and used. Specifically, the surfactant may be mixed in a dry manner, or dissolved in a solvent and then mixed in a solution state, or the surfactant may be melted and then mixed.

Among them, for example, the surfactant may be mixed in a solution state in which it is dissolved in a solvent. At this time, as the solvent, any kind of inorganic solvent or organic solvent can be used without limitation. Considering the ease of the drying process and the cost of the solvent recovery system, water is most suitable.

In addition, a method of putting the surfactant and the polymer in a reaction tank and mixing the solution, or a method of putting the polymer in a mixer and spraying the solution, a method of continuously charging and mixing a polymer and a solution into a continuously operated mixer, and the like can be used.

Meanwhile, according to an embodiment of the present disclosure, a step of neutralizing at least a part of the acidic group of the polymer (step 2), and a step of micronizing the polymer in the presence of a surfactant (step 3) can be performed sequentially, alternately, or simultaneously.

That is, a neutralizing agent is added to the polymer to neutralize the acidic group first, and then a surfactant may be added to the neutralized polymer to micronize the polymer mixed with the surfactant (performed in the order of step 2 -> step 3), a neutralizing agent and a surfactant may be simultaneously added to the polymer to neutralize and micronize the polymer (steps 2 and 3 are performed simultaneously). Alternatively, the surfactant may be added first and the neutralizing agent may be added later (performed in the order of step 3->step 2). Alternatively, the neutralizing agent and the surfactant may be alternately added. Alternatively, a surfactant is first added and micronized, then a neutralizing agent is added and neutralized, and further a surfactant is further added to the neutralized hydrogel polymer to perform the micronizing step

Meanwhile, it may be desirable to provide a certain time difference between the charging of the neutralizing agent and the micronizing process in order to evenly neutralize the entire polymer.

At least a part to a significant amount of the surfactant may be present on the surface of the super absorbent polymer.

Here, the surfactant being present on the surface of the super absorbent polymer means that at least a part or a significant amount of the surfactant is adsorbed or bonded onto the surface of the super absorbent polymer. Specifically, the surfactant may be physically or chemically adsorbed on the surface of the super absorbent polymer. More specifically, the hydrophilic functional group of the surfactant may be physically adsorbed to the hydrophilic part of the surface of the super absorbent polymer by intermolecular force such as dipole-dipole interaction. In this manner, the hydrophilic portion of the surfactant is physically adsorbed on the surface of the super absorbent polymer particles to surround the surface, and the hydrophobic portion of the surfactant is not adsorbed to the surface of the resin particles, so that the resin particles can be coated with a surfactant in the form of a kind of micelle structure. This is because the surfactant is not added during the polymerization process of the water-soluble ethylenically unsaturated monomer, but added in the micronizing process after polymer formation. It can faithfully perform its role as a surfactant as compared to the case where the surfactant is added during the polymerization process and the surfactant is present inside the polymer, and pulverization and aggregation occur at the same time to obtain particles with a large surface area in the form in which fine particles are aggregated.

According to an embodiment of the present disclosure, the step of micronizing the polymer to produce hydrous super absorbent polymer particles may be performed two or more times.

According to an embodiment of the present disclosure, the micronizing step is performed by a micronizing device, wherein the and micronizing device may include a body portion including a transfer space in which the polymer is transferred inside; a screw member rotatably installed inside the transfer space to move the polymer; a driving motor that provides a rotational driving force to the screw member; a cutter member installed in the body portion to pulverize the polymer; and a perforated plate in which the polymer pulverized by the cutter member is discharged to the outside of the body, and a large number of holes are formed in the plate. At this time, the hole size provided in the perforated plate of the micronizing device may be 1 mm to 20 mm, or 5 mm to 15 mm, or 5 mm to 12 mm.

According to one embodiment of the present disclosure, the primary and secondary micronizing steps are performed by primary and secondary micronizing devices, respectively. The primary and secondary micronizing devices may include a body portion including a transfer space in which the polymer is transferred inside; a screw member rotatably installed inside the transfer space to move the polymer; a driving motor that provides a rotational driving force to the screw member; a cutter member installed in the body portion to pulverize the polymer; and a perforated plate in which the polymer pulverized by the cutter member is discharged to the outside of the body portion, and a large number of holes are formed in the plate.

Hole sizes of the perforated plates respectively provided in the primary and secondary micronizing devices may be the same as or different from each other.

Meanwhile, according to one embodiment of the present disclosure, in order to facilitate pulverization, the hole size provided in the perforated plate of the secondary micronizing device is preferably smaller than the hole size of the perforated plate provided in the perforated plate of the primary micronizing device. For example, the hole size provided in the perforated plate of the primary micronizing device is 1 mm to 6 mm, and the hole size provided in the perforated plate of the secondary micronizing device may be 0.5 mm to 6 mm.

In this manner, when the polymer mixed with the surfactant is micronized using a micronizing device, a smaller particle size distribution is realized and subsequent drying and pulverization processes can be performed under milder conditions, thereby capable of improving the physical properties of the super absorbent polymer while preventing the generation of fines.

### Step 4: Drying Step

Next, a step of drying the neutralized and micronized polymer to produce dry super absorbent polymer particles (step 4) is performed.

The above step is a step of neutralizing at least a part of the acidic group of the polymer, micronizing the polymer in the presence of a surfactant, and drying the moisture of the hydrous super absorbent polymer particles, which is the resulting polymer.

In a conventional method for preparing a super absorbent polymer, the drying step is generally performed until the moisture content of the super absorbent polymer becomes less than 10 wt.%, but according to one embodiment of the present disclosure, the drying step is performed so that the moisture content of the super absorbent polymer becomes 10 wt.% or more, for example, about 10 to about 20 wt.%, or about 10 to about 15 wt.%. However, the present disclosure is not limited thereto.

For this purpose, the temperature in the dryer used in the drying step is about 150°C or less, for example, about 80°C to about 150°C, and drying can be performed at a relatively low temperature. When the temperature inside the dryer is too low, the drying time may be too long, and when the drying temperature is too high, a super absorbent polymer having a moisture content lower than a desired moisture content can be obtained.

At this time, the drying may be performed by a moving type. Such moving type drying is divided into a stationary drying and the presence/absence of flow of material during drying.

The moving type drying refers to a method of drying the product while mechanically stirring it. At this time, the direction in which the hot air passes through the material may be the same as or different from the circulation direction of the material. Alternatively, the material can be circulated inside the dryer, and the heat-transfer fluid (heat-transfer oil) may be passed through a separate pipe outside the dryer to dry the material.

Meanwhile, the stationary drying refers to a method in which a material to be dried is stopped at the bottom, such as a perforated iron plate through which air can pass, and hot air passes through the material from the bottom to the top to dry it.

Therefore, it is preferable to dry the hydrous super absorbent polymer by a moving type drying method in terms of being able to complete uniform drying within a short time that is to be dried in the above step.

As a device capable of drying by such a moving type drying method, a horizontal-type mixer, a rotary kiln, a paddle dryer, a steam tube dryer, or a generally used moving type dryer can be used.

### Step 5: Pulverizing Step

Next, a step of pulverizing the dried super absorbent polymer particles to produce super absorbent polymer particles is performed.

Specifically, the pulverizing step can be performed to pulverize the dried super absorbent polymer particles to have a particle size of a normal particle level, that is, a particle size of 150 µm to 850 µm.

The pulverizing device used for this purpose may be specifically a vertical pulverizer, a turbo cutter, a turbo grinder, a rotary cutter mill, a cutter mill, a disc mill, a shred crusher, a crusher, a chopper or disc cutter, or the like, but is not limited the examples described above.

Alternatively, as the mill, a pin mill, a hammer mill, a screw mill, a roll mill, a disc mill, or a jog mill can be used, but is not limited to the examples described above.

Meanwhile, in the preparation method of the present disclosure, the super absorbent polymer particles having a smaller particle size distribution than in the conventional chopping step can be realized in the micronizing step. When performing moving type drying, since the moisture content after drying is maintained relatively high at 10 wt.% or more, it is possible to form a super absorbent polymer having a very high content of a normal particle size of 150 µm to 850 µm even if the pulverization is performed under mild conditions with less pulverization force, and fines generation rate can be greatly reduced.

The super absorbent polymer particles produced as described above may contain super absorbent polymer particles having a particle size of 150 *µ*m to 850 ,um, that is, normal particles, in an amount of 80 wt.% or more, 85 wt.% or more, 89 wt.% or more, 90 wt.% or more, 92 wt.% or more, 93 wt.% or more, 94 wt.% or more, or 95 wt.% or more with respect to the total weight.

The particle size of the polymer particles may be measured according to the European Disposables and Nonwovens Association standard EDANA WSP 220.3.

Further, the super absorbent polymer particles may contain fines having a particle size of less than 150 *µ*m in an amount of about 20 wt.% or less, or about 18 wt.% or less, or about 15 wt.% or less, or about 13 wt.% or less, or about 12 wt.% or less, or about 11 wt.% or less, or about 10 wt.% or less, or about 9 wt.% or less, or about 8 wt.% or less, or about 5 wt.% or less with respect to the total weight. This is in contrast to the case having fines of greater than about 20 wt.% to about 30 wt.% when the super absorbent polymer is prepared according to a conventional preparation method.

### Additional step

After the step of pulverizing the super absorbent polymer particles, the method may further include a step of classifying the pulverized super absorbent polymer particles in accordance with particle sizes.

Further, the method may further include a step of forming a surface crosslinked layer on at least a part of the surface of the super absorbent polymer particles in the presence of a surface crosslinking agent after pulverizing and/or classifying the super absorbent polymer particles. Through the above step, the crosslinked polymer contained in the super absorbent polymer particles may be further crosslinked via the surface crosslinking agent to form a surface crosslinked layer on at least a part of the surface of the super absorbent polymer particles.

As the surface crosslinking agent, any surface crosslinking agent conventionally used in the production of super absorbent polymers may be used without particular limitation. For example, the surface crosslinking agent may include at least one polyol selected from the group consisting of ethylene glycol, propylene glycol, 1,3-propanediol, 1,4-butanediol, 1,6-hexanediol, 1,2-hexanediol, 1,3-hexanediol, 2-methyl-1,3-propanediol, 2,5-hexanediol, 2-methyl-1,3-pentanediol, 2-methyl-2,4-pentanediol, tripropylene glycol and glycerol; at least one carbonate-based compound selected from the group consisting of ethylene carbonate, propylene carbonate and glycerol carbonate; an epoxy compound, such as ethylene glycol diglycidyl ether; an oxazoline compound such as oxazolidinone; a polyamine compound; an oxazoline compound; mono-, di- or polyoxazolidinone compounds; or a cyclic urea compound; and the like.

Specifically, one or more, or two or more, or three or more of the above-mentioned surface crosslinking agents can be used as the surface crosslinking agent. For example, ethylene carbonate-propylene carbonate (ECPC), propylene glycol and/or glycerol carbonate can be used.

Such a surface crosslinking agent can be used in an amount of about 0.001 to about 5 parts by weight with respect to 100 parts by weight of the superabsorbent polymer particles. For example, the surface crosslinking agent may be used in an amount of 0.005 parts by weight or more, or 0.01 parts by weight or more, or 0.05 parts by weight or more, or 5 parts by weight or less, or 4 parts by weight or less, or 3 parts by weight or less with respect to 100 parts by weight of the super absorbent polymer particles. By adjusting the content range of the surface crosslinking agent to the above-mentioned range, a super absorbent polymer having excellent absorption properties can be prepared.

Further, the step of forming the surface crosslinking layer can be performed by adding an inorganic material to the surface crosslinking agent. That is, the step of further crosslinking the surface of the super absorbent polymer particles in the presence of the surface crosslinking agent and the inorganic material to form a surface crosslinking layer can be performed.

As such an inorganic material, at least one inorganic material selected from the group consisting of silica, clay, alumina, silica-alumina composite, titania, zinc oxide, and aluminum sulfate can be used. The inorganic material can be used in powder form or liquid form, and particularly, it can be used as alumina powder, silica-alumina powder, titania powder, or nano silica solution. Further, the inorganic material can be used in an amount of about 0.001 to about 1 part by weight with respect to 100 parts by weight of the super absorbent polymer particles.

With regard to the method of mixing the surface crosslinking agent with the super absorbent polymer, its constitution is not limited. For example, a method of adding and mixing the surface crosslinking agent and the super absorbent polymer powder in a reactor, a method of spraying the surface crosslinking agent onto the super absorbent polymer composition, or a method of continuously charging the super absorbent polymer composition and the surface crosslinking agent into a mixer which is continuously operated, or the like, can be used.

When the surface crosslinking agent and the super absorbent polymer composition are mixed, water and methanol may be further mixed together and added. When water and methanol are added, there is an advantage that the surface crosslinking agent may be appropriately adjusted in order to induce uniform dispersion of the surface crosslinking agent, prevent aggregation of the super absorbent polymer composition, and at the same time, optimize the surface penetration depth of the crosslinking agent.

The surface crosslinking process can be performed at a temperature of about 80°C to about 250°C. More specifically, the surface crosslinking process can be performed at a temperature of about 100°C to about 220°C, or about 120°C to about 200°C for about 20 minutes to about 2 hours, or about 40 minutes to about 80 minutes. When the above-mentioned surface crosslinking process conditions are satisfied, the surface of the super absorbent polymer particles may be sufficiently crosslinked to increase absorbency under pressure.

A means for raising the temperature for the surface crosslinking reaction is not particularly limited. Heating may be performed by providing a heating medium or by directly providing a heat source. The type of the heating medium applicable herein may be a hot fluid such as steam, hot air, hot oil, or the like, but is not limited thereto. Further, the temperature of the heating medium provided can be appropriately selected considering the means of the heating medium, the temperature-raising rate, and the temperature-raising target temperature. Meanwhile, as the heat source provided directly, an electric heater or a gas heater may be used, but is not limited to the above-described examples.

According to one embodiment of the present disclosure, after the forming of the surface crosslinked layer on at least a part of the surface of the super absorbent polymer particles, a cooling step of cooling the super absorbent polymer particles on which the surface crosslinked layer is formed, the method may further comprise any one or more steps of: a cooling step of cooling the super absorbent polymer particles on which the surface crosslinked layer is formed; a water addition step of adding water to the super absorbent polymer particles on which the surface crosslinked layer is formed; and a post-treatment step of adding an additive to the super absorbent polymer particles on which the surface crosslinked layer is formed. At this time, the cooling step, the water addition step, and the post-treatment step may be performed simultaneously.

The additive added in the post-treatment step may be a liquid permeability improver, an anti-caking agent, a fluidity improver, an antioxidant, and the like, but the present disclosure is not limited thereto.

By selectively performing the cooling step, the water addition step, and the post-treatment step, the moisture content of the final super absorbent polymer can be improved, and a higher quality super absorbent polymer product can be produced.

According to yet another embodiment of the present disclosure, there is provided a super absorbent polymer prepared by the above preparation method.

The super absorbent polymer prepared by the above preparation method has a fast vortex time and a low fines content, and also has a centrifuge retention capacity (CRC) and absorbency under pressure (AUP), which are general absorption properties, in a level equal to or higher than those of a super absorbent polymer prepared by a conventional method.

Further, it is possible to provide a super absorbent resin that can have a uniform particle size distribution by narrowing the particle size distribution and is excellent in liquid permeability and rewet properties by lowering the content of water-soluble components.

Hereinafter, preferred examples are presented to aid in understanding of the invention. However, the following examples are for illustrative purposes only, and the scope of the invention is not intended to be limited thereby.

### <Example>

An acrylic acid (AA) aqueous solution was used as the monomer component.

In order to remove dissolved oxygen in the monomer aqueous solution, nitrogen gas was used to perform nitrogen purge at 1 L/min at a temperature of 5°C for about 1 hour.

As an internal crosslinking agent component, P-30 (Pentaerythritol diallyl ether) was used by mixing about 3500 ppmw relative to the acrylic acid.

As an initiator component, about 600 ppmw (relative to acrylic acid) of VA-086 as an azo-based initiator, and about 40 ppmw (relative to acrylic acid) of hydrogen peroxide were mixed so as to be supplied in the form of a separate aqueous solution.

As a reducing agent component, bout 150 ppmw of ascorbic acid (relative to acrylic acid) and about 1.5 ppmw of iron sulfate (FeSO₄) (relative to acrylic acid) were mixed so as to be supplied in the form of a separate aqueous solution.

The concentrations of solutes in each aqueous solution are summarized separately in Table 1 below.

**[Table 1]**

| Category | Monomer aqueous solution (wt.%) | | Initiator *M/U solution (wt.%) | | Accelerator *M/U solution (wt.%) | |
|---|---|---|---|---|---|---|
| | AA | P-30 | VA-086 | H₂O₂ | Ascorbic acid | FeSO₄ |
| Example 1 | 30.83 | 0.11 | 1.30 | 0.09 | 0.50 | 0.010 |
| Example 2 | 31.72 | 0.11 | 0.65 | 0.04 | 0.25 | 0.005 |
| Example 3 | 33.65 | 0.12 | 0.33 | 0.02 | 0.12 | 0.002 |
| Example 4 | 31.72 | 0.11 | 0.65 | 0.04 | 0.25 | 0.005 |
| Example 5 | 31.72 | 0.11 | 0.65 | 0.04 | 0.25 | 0.005 |

| | | | | | | |
|---|---|---|---|---|---|---|
| *Make Up | | | | | | |

The monomer aqueous solution, the initiator aqueous solution, and the reducing agent aqueous solution were all supplied through respective transfer lines, and just before reaching the reactor, the initiator transfer line and the reducing agent transfer line were configured so as to be sequentially combined with the monomer transfer line.

The supply process conditions of the monomer transfer line, the initiator transfer line and the reducing agent transfer line are summarized in Table below.

**[Table 2]**

| Category | Monomer | Initiator | Accelerator | Main diameter* | Nozzle diameter** |
|---|---|---|---|---|---|
| | Flow rate (kg/hr) | Flow rate (kg/hr) | Flow rate (kg/hr) | (m) | (m) |
| Example 1 | 10701.6 | 152.5 | 149.0 | 0.0779 | 0.005 |
| Example 2 | 10403.0 | 302.6 | 297.4 | 0.0779 | 0.005 |
| Example 3 | 9805.8 | 602.8 | 594.4 | 0.0779 | 0.005 |
| Example 4 | 10403.0 | 302.6 | 297.4 | 0.0779 | 0.010 |
| Example 5 | 10403.0 | 302.6 | 297.4 | 0.0390 | 0.005 |

| | | | | | |
|---|---|---|---|---|---|
| * Monomer transfer line diameter ** Initiator transfer line and reducing agent transfer line diameter (circular) | | | | | |

Under the above conditions, a monomer aqueous solution, an initiator, and a reducing agent were supplied (supply amount) to a reactor for 1 hour, the reaction was initiated and the polymerization reaction proceeded for about 6 hours at a temperature of about 90°C to form a crosslinked polymer.

The obtained crosslinked polymer was dried/pulverized to obtain a powder form, and the content of unreacted monomers in the polymer was analyzed for the sample according to the EDANA NWSP 210.0.R2(15).

The above contents are summarized in Table below.

**[Table 3]**

| Catego ry | Monom er flow speed (m/s) | Initial or flow speed (m/s) | Accelerat or flow speed (m/s) | Speed ratio of initiator/mono mer | Speed ratio of accelerator/in itiator | Content of unreacted monomer (ppmw) |
|---|---|---|---|---|---|---|
| Exampl e 1 | 0.62 | 2.16 | 2.11 | 3.5 | 3.4 | 10000 |
| Exampl e 2 | 0.61 | 4.28 | 4.21 | 7.1 | 6.9 | 2000 |
| Exampl e 3 | 0.57 | 8.53 | 8.41 | 14.9 | 14.7 | 800 |
| Exampl e 4 | 0.61 | 1.07 | 1.05 | 1.8 | 1.7 | 13000 |
| Exampl e 5 | 2.43 | 4.28 | 4.21 | 1.8 | 1.7 | 11000 |

Referring to Table 2, it can be confirmed that when a specific rate ratio is satisfied as in Examples 2 and 3, the content of unreacted monomer is greatly reduced.

This is considered to be because, according to Bernoulli's principle, the fluid side with a relatively low speed (monomer transfer line) is increased in pressure, the fluid side with a relatively high speed (initiator transfer line) is reduced in pressure, so that rapid diffusion occurs instantaneously, and the monomer component and the initiator component are quickly and uniformly mixed with each other.

## Claims

1. A method for preparing a super absorbent polymer, comprising the steps of:
step 1: polymerizing a monomer composition containing a water-soluble ethylenically unsaturated monomer having an acidic group, an internal crosslinking agent, and a polymerization initiator to form a polymer in which the water-soluble ethylenically unsaturated monomer having an acidic group and the internal crosslinking agent are subjected to a crosslinking polymerization;
step 2: neutralizing at least a part of the acidic group of the polymer to form a hydrogel polymer;
step 3: micronizing the polymer in the presence of a surfactant; and
step 4: drying the neutralized and micronized polymer to prepare dried super absorbent polymer particles,
wherein in the step of forming the polymer, a first monomer composition containing the monomer and the internal crosslinking agent is transferred through a monomer transfer line, the polymerization initiator is transferred through an initiator transfer line, and the monomer transfer line and the initiator transfer line are combined just before being charged into the polymerization reactor, and the first monomer composition and the initiator are mixed to form a second monomer composition.

2. The method for preparing a super absorbent polymer according to claim 1 wherein:
the step of forming the polymer is performed in a batch type reactor.

3. The method for preparing a super absorbent polymer according to claim 1 wherein:
the step 2 and the step 3 are performed sequentially, simultaneously, or alternately.

4. The method for preparing a super absorbent polymer according to claim 1 wherein:
in the step of combining the monomer transfer line and the initiator transfer line, the ratio (speed ratio) of the supply speed (m/s) of the initiator supplied from the initiator transfer line to the supply speed (m/s) of the first monomer mixture supplied from the monomer transfer line is 3.6 or more.

5. The method for preparing a super absorbent polymer according to claim 1 wherein:
the second monomer composition further comprises a reducing agent, the reducing agent is supplied together with the initiator through the initiator transfer line, or is supplied through a separate reducing agent transfer line, and
the ratio (speed ratio) of the supply speed (m/s) of the reducing agent to the supply speed (m/s) of the first monomer mixture supplied from the monomer transfer line is also 3.5 or more.

6. The method for preparing a super absorbent polymer according to claim 1 wherein:
in the step of combining the monomer transfer line and the initiator transfer line, a ratio (flow rate ratio) of the supply flow rate (kg/hr) of the initiator supplied from the initiator transfer line to the supply flow rate (kg/hr) of the first monomer mixture supplied from the monomer transfer line is 0.01 to 0.1.

7. The method for preparing a super absorbent polymer according to claim 1 wherein:
the step of drying the neutralized and micronized polymer is performed by a moving type drying.

8. The method for preparing a super absorbent polymer according to claim 7 wherein:
the moving type drying is performed using a horizontal-type mixer, a rotary kiln, a paddle dryer, or a steam tube dryer.

9. The method for preparing a super absorbent polymer according to claim 1 wherein:
the step of drying the neutralized and micronized polymer is performed at a temperature of 150°C or less.

10. The method for preparing a super absorbent polymer according to claim 1 wherein:
the dried super absorbent polymer particles obtained by drying the neutralized and micronized polymer has a moisture content of 10 to 30 wt.%.

11. The method for preparing a super absorbent polymer according to claim 1 wherein:
at least a part of the surfactant is present on the surface of the hydrogel polymer.

12. The method for preparing a super absorbent polymer according to claim 1 wherein:
the surfactant comprises at least one selected from the group including a compound represented by the following Chemical Formula 2, a salt thereof, a compound represented by the following Chemical Formula 3, and a salt thereof: 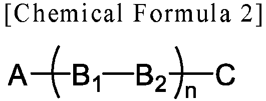
wherein, in Chemical Formula 2,
A is an alkyl having 5 to 21 carbon atoms,
B₁ is -OCO-, -COO-, or -COOCH(R₁)COO-,
B₂ is -CH₂-, -CH₂CH₂-, -CH(R₂)-, -CH=CH-, or -C≡C-,
where, R₁ and R₂ are each independently an alkyl having 1 to 4 carbon atoms,
n is an integer of 1 to 3, and
C is a carboxyl group, 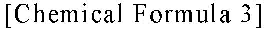 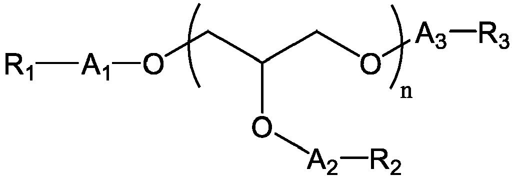
wherein, in Chemical Formula 3,
A₁, A₂ and A₃ are each independently a single bond, carbonyl, 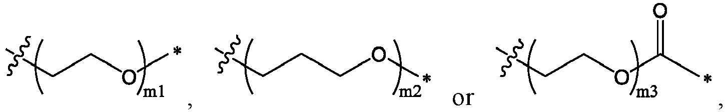 provided that one or more thereof are carbonyl or 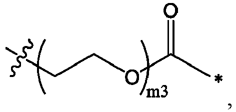 where m1, m2 and m3 are each independently an integer of 1 to 8, each 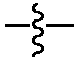 is connected to an adjacent oxygen atom, and 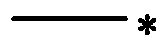 is connected to adjacent R₁, R₂ an R₃, respectively,
R₁, R₂ and R₃ are each independently hydrogen, a linear or branched alkyl having 6 to 18 carbon atoms, or a linear or branched alkenyl having 6 to 18 carbon atoms, and
n is an integer of 1 to 9.

13. The method for preparing a super absorbent polymer according to claim 1 wherein:
the super absorbent polymer particles contain 89 wt.% or more of super absorbent polymer particles having a particle size of 150 µm to 850 µm with respect to the total weight of the super absorbent polymer particles.

14. The method for preparing a super absorbent polymer according to claim 1 wherein:
the super absorbent polymer particles contain 20 wt.% or less of super absorbent polymer particles having a particle size of less than 150 µm with respect to the total weight of the super absorbent polymer particles.

15. The method for preparing a super absorbent polymer according to claim 1 wherein:
after the step of drying the neutralized and micronized polymer to prepare super absorbent polymer particles, the method further comprises pulverizing the super absorbent polymer particles.

16. The method for preparing a super absorbent polymer according to claim 15 wherein:
after the step of further pulverizing the super absorbent polymer particles, the method further comprises classifying the pulverized super absorbent polymer particles in accordance with particle sizes.

17. The method for preparing a super absorbent polymer according to claim 1 or 16, further comprising:
forming a surface crosslinked layer on at least a part of the surface of the super absorbent polymer particles.

18. The method for preparing a super absorbent polymer according to claim 17 wherein:
after the step of forming a surface crosslinked layer on at least a part of the surface of the super absorbent polymer particles,
the method further comprises any one or more steps of: a cooling step of cooling the super absorbent polymer particles on which the surface crosslinked layer is formed; a water addition step of adding water to the super absorbent polymer particles on which the surface crosslinked layer is formed; and a post-treatment step of adding an additive to the super absorbent polymer particles on which the surface crosslinked layer is formed.

19. The method for preparing a super absorbent polymer according to claim 18 wherein:
the cooling step, the water addition step, and the post-treatment step are performed simultaneously.

20. A super absorbent polymer prepared by the preparation method according to claim 1.
